# EUROPEAN PATENT APPLICATION

(11) **EP 3 982 321 A1**
(43) Date of publication of application: **13.04.2022**
(21) Application number: 19932528.3
(22) Date of filing: 13.12.2019
(51) Int. Cl.: G06T 1/40, G06T 7/11, G06K 9/50

(54) **SYSTEM FOR PROCESSING RADIOGRAPHIC IMAGES AND OUTPUTTING THE RESULT TO A USER**

(30) Priority: 10.06.2019 RU 2019118035
(71) Applicant: Obshchestvo S Ogranichennoj Otvetstvennost'Yu "Medicinskie Skrining Sistemy", Kaluzhskaya obl., g. Kaluga, 248000 (RU)
(72) Inventor: CHERNIN, Igor' Il'ich, Kaluga, 248000 (RU); CHERNIN, Vladimir Il'ich, Kaluga, 248000 (RU); CHERNIN, Stanislav Igorevich, Kaluga, 248000 (RU); NIKOLAEV, Nikita Alekseevich, St.Petersburg, 198206 (RU); NIKITIN, Evgenij Dmitrievich, St.Petersburg, 193168 (RU); KAPNINSKIJ, Artyom Aleksandrovich, Kaluga, 248000 (RU)
(74) Representative: Benatov, Samuil Gabriel
(86) International application number: PCT/RU2019/000947
(87) International publication number: WO 2020/251396

(57) **Abstract**

The invention relates to the field of computer engineering for processing images. The technical result consists in increasing the accuracy of finding and classifying a similar object when processing radiographic images and outputting the result of the processing to a user. The technical result is achieved by: downloading files of a radiographic image which comprise metadata including information about the object or subject of the image and information about the image itself; encrypting the downloaded files if the above-mentioned files comprise personal data about a person; decrypting the above-mentioned, encrypted, downloaded files; and processing the radiographic image, wherein, as a result of the processing, the following occurs: finding and capturing a relevant region of the radiographic image; removing noise from the captured, relevant region of the radiographic image, wherein a region with a found object is meant by a relevant region of the radiographic image; compressing or unzipping a previously processed radiographic image; and finding a similar object in two previously processed images, and processing said object.

## Description

### TECHNICAL FIELD OF THE INVENTION

The invention relates to the field of computer technology for image processing, and can be used in the field of medicine, for example, for the diagnostics of oncological diseases, or in the field of industry, for example, for the detection of hidden defects of an industrial facility.

### BACKGROUND OF THE INVENTION

There are currently many systems designed to process images to detect, for example, oncological diseases. One example of such systems is a system for diagnosing a stomach cancer by using a convolutional neural network, which is described in CN 107368670 A. This solution involves obtaining image data of a slice of a normal stomach tissue and a slice of a tissue under study, comparing the image data, and drawing a conclusion based on said comparison. As a result of said comparison, the neural network remembers the result and uses it in the future, i.e., it is trainable.

However, the known solution has disadvantages. One of the disadvantages of the known solution consists in the low accuracy of processing X-ray images and outputting the processing result to a user, since the known system does not involve removing noises from a captured relevant region of an X-ray image, as well as comparing at least two images of the tissue under study to find a similar object, the two images being two pictures of different projections of the same tissue under study or the same industrial facility under study. Moreover, the known solution does not ensure the confidentiality of transmitted data by encrypting user personal data when they are found on the X-ray image, the personal data meaning any data that can uniquely identify their owner.

### SUMMARY OF THE INVENTION

The objective of the invention is to eliminate the above-mentioned disadvantages.

The technical result is an increase in the accuracy of finding and classifying a similar object when processing X-ray images and outputting the processing result to a user, while ensuring the confidentiality of transmitted data by encrypting user personal data when they are used or found on an X-ray image, the personal data being any data that can uniquely identify their owner.

To achieve this technical result, a system for processing an X-ray image and outputting a processing result to a user is provided. The system comprises: an X-ray image input unit configured to download X-ray image files containing metadata comprising information about an object or subject of an X-ray image and information about the X-ray image itself and transmit the downloaded files to an X-ray image pre-processing unit, as well as configured to encrypt the downloaded files if the files contain personal data of a person; the X-ray image pre-processing unit configured to decrypt the encrypted downloaded files, process the X-ray image and transmit the pre-processed X-ray image to a compressing or unzipping unit, wherein said processing comprises: finding and capturing a relevant region of the X-ray image, removing noises from the captured relevant region of the X-ray images, the relevant region of the X-ray image being a region with the found object; the compressing or unzipping unit configured to compress or unzip the pre-processed X-ray image for further transmission to an object finding unit; a unit for finding at least one similar object, configured to find the at least one similar object in at least two pre-processed images, process the at least one similar object and transmit the found and processed object to a data processing unit, said finding and processing comprise: finding the similar object in the images and superposing variable coordinates, distances and metrics on the images, the two pre-processed images being two pictures of different projections of the same human tissue or industrial facility under study; the data processing unit configured to process the data by using a trainable neural network and transmit the processed data to a data post-processing unit, wherein said processing by using the neural network and its training comprise identifying patterns of the found and processed objects of a set of X-ray images and then identifying the region in the found and processed object based on the identified patterns; the data post-processing unit configured to post-process and transmit the data to a data storage unit, a display unit and a data interpretation unit, wherein said post-processing comprises calculating a result of matching physical parameters of the identified region with the identified patterns in percentage terms and classifying the identified region; the data interpretation unit configured to generate, encrypt and transmit interpreted data to the display unit, wherein the data interpretation unit is connected to a database of the data storage unit, the database comprising data of all classifications of the identified regions, wherein said interpreting comprising determining an influence of the found objects of various classes on final-recommendation generation and highlighting regions of the image which have most influenced a model prediction consisting in that this is an object of a certain class; and the display unit configured to decrypt the encrypted interpreted data and output the decrypted interpreted data to at least one user.

Additionally, said finding and processing further comprise building a three-dimensional display of a shape of the found object, and said superposing the variable coordinates, distances and metrics on the images is performed in accordance with the three-dimensional display.

Additionally, the trainable neural network comprises: a convolution component configured to transform the original X-ray image into a map of variables which encodes information about objects in the original X-ray image and transmit the map of variables to a region proposal network (RPN) component; the RPN component configured to calculate predictions of relevant objects on the map of variables and transmit the map of variables and the calculated predictions to a classification component; the classification component configured to calculate a probability that the object found in the X-ray image is a background object, and transmit classified data to a regression component and an aggregation component; the regression component configured to determine an exact location of the found object in the image and transmit processed data to the aggregation component; and the aggregation component configured to aggregate information of the data obtained from the classification component and the regression component, wherein the aggregation yields summary data of a type of the found object.

Additionally, the X-ray image has a format that may be at least one of: DICOM, JPEG, JPG, GIF, PNG.

Additionally, the X-ray image is an X-ray picture of human organs or tissues, or an X-ray picture of an industrial facility.

Additionally, the object has a physical parameter that is a kind of the object.

It should be obvious that both the previous summary and the following detailed description are given by way of example and explanation only and are not limitations of the present invention.

### BRIEF DESCRIPTION OF THE DRAWING

FIG. 1 schematically shows a claimed system for processing X-ray images and outputting a processing result to a user.
FIG. 2 shows a block diagram of steps for processing X-ray images.

### DETAILED DESCRIPTION OF THE INVENTION

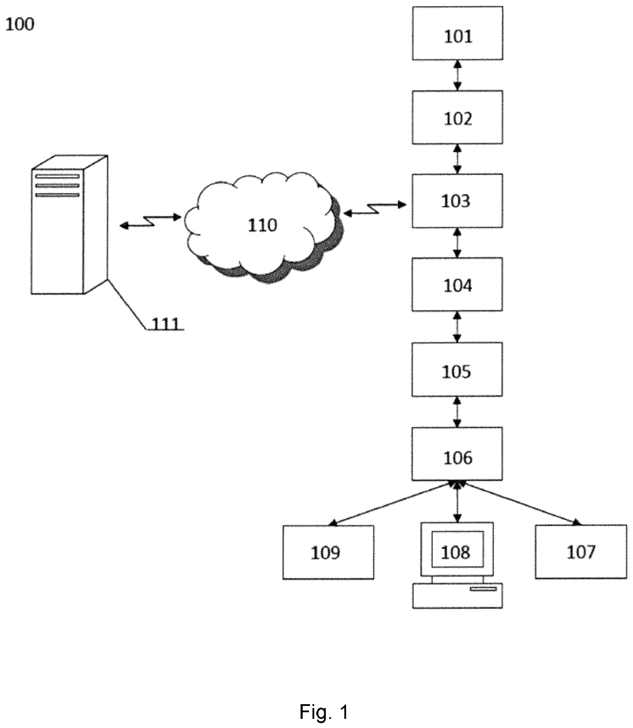

A claimed system for processing X-ray images and outputting a processing result to a user is schematically shown in FIG. 1. The system comprises a data input unit 101, a data pre-processing unit 102, a compressing or unzipping unit 103, a unit 104 for finding at least one similar object, a data processing unit 105, a data post-processing unit 106, a data storage unit 107, a display and output unit 108, a data interpretation unit 109, a data network 110, a remote server 111. The data input unit 101, the data pre-processing unit 102, the compressing or unzipping unit 103, the unit 104 for finding the at least one similar object and the data processing unit 105 are, respectively, connected in series. The data post-processing unit 106 is connected to the data processing unit 105, the data interpretation unit 109, the data storage unit 107, and the display and output unit 108. The units 101, 102, 103, 104, 105, 106, 107, 108, 109 are implemented on a user computing device. The computing device refers to any device that comprises at least a memory, a processor, and a video card having a capacity of at least 4 GB.

The X-ray image input unit 101 downloads files in DICOM, JPEG, JPG, GIF, or any other format of X-ray images comprising object and X-ray image meta-information, as well as the digital pictures themselves, and transmits the downloaded files to the X-ray image pre-processing unit 102. The downloaded files are transmitted to the remote server 111. Depending on a type of an object under study and/or disease and/or pathology and/or deformation, as well as on a type of equipment by which a picture was obtained (an X-ray machine, a digital microscope, a computed tomography scanner, etc.), 1 to 12 different projections of the object are used. In this case, all the above-mentioned operations may be performed locally on the user computing device and/or on the remote server. Additionally, if personal data are found in the downloaded files, the unit 101 implements the cryptographic protection of the personal data by means of encryption. The cryptographic protection is carried out either using third-party solutions, depending on the legislation of the country in which the system for processing the X-ray images and outputting the processing result to the user is used, or by embedding cryptographic protection software in the unit 101.

The data pre-processing unit 102 decrypts the data encrypted by the unit 101, as well as pre-process the data using the meta-information from the files to determine a side (left, right (if there is a symmetric object)) and type of a projection and transmits the pre-processed X-ray images to the compressing/unzipping unit 103. Each picture undergoes automatic pre-processing, during which artifacts (e.g., the presence of extraneous glow, extraneous inclusions and any other elements interfering with the detection of a main pathology in the picture) are removed from the picture, as well as a relevant region is found in the picture with the subsequent removal of identified excess parts. As a result of this procedure, each picture acquires a standardized dimension that differs depending on a type of diagnostics. During the pre-processing, the relevant region of the X-ray image is found and captured, and noises are removed from the captured relevant region of the X-ray image. The relevant area of the X-ray image is a region of interest with a found object.

The compressing or unzipping unit 103 compresses or unzips the pre-processed X-ray images for their further transmission to the object finding unit 104. The main function of the unit 103 is to compress and unzip the results of the data pre-processing unit 102 in the form of prepared DICOM, JPEG, JPG, PNG files, without loss of quality during the compressing-unzipping procedures. Based on the result of the pre-processing, the compressing or unzipping procedure is implemented using the LZMA SDK library, where the LZMA SDK library provides X-ray image compressing or unzipping. In this case, the unit 103 is communicatively connected through the data network 110 with the remote server 111.

The unit 104 for finding the at least one similar object is configured to find the at least one similar object in at least two pre-processed images, process the at least one similar object and transmit the found and processed object to the data processing unit 105. The main purpose of the unit 104 is to identify the same object on different projections, for example, an organ in case of diagnostics based on CT, MRI pictures. When labeling the data, each object is assigned a class to which the object belongs (e.g., a mass lesion, or a benign tumor, or a malignant tumor, etc.). Said labeling involves superposing variable coordinates, distances, and metrics on the images. The unit draws conclusions about the following by verifying the objects on the projections: finding the same object on the different projections, building the three-dimensional display of the object shape, checking for the benignity/malignancy of the object detected on both projections, generating an additional set of variables based on the detected objects on the different projections in the form of coordinates, distances, and metrics.

The data processing unit 105 is configured to process the data by means of a trainable neural network and transmit the processed data to the data post-processing unit 106. In this unit, each picture is fed to the input of the pretrained neural network. Training is carried out using two sources - public sets of data with labels at the level of object kind, pathology and/or disease (coordinates, disease type and tumor type), physical data of the object under study (density standards, permissible errors), as well as a proprietary set of data with labels at the level of an organ (pathology/absence of pathology) or the object. After the architecture of the neural network is selected, the neural network is trained using the labeled data. At each step, the neural network calculates predictions for one or more pictures, these predictions are compared with the ground truth, and a loss function value is calculated (how much the neural network has been mistaken in detecting, determining the class and location of the object). Further, by using the gradient descent method and the backpropagation algorithm, all weights of the neural network change in accordance with a selected learning rate parameter in the direction opposite to the calculated gradient to minimize an error for the current picture(s). This step is repeated many times, and the learning process results in the neural network weights converging to the optimal ones. As a result of the above-mentioned procedures, each object is subjected to classification (for example, mass lesion, benign tumor, malignant tumor, etc.). The optimal hyperparameters and system parameters which define, among other things, a percentage of introduced errors are set both by the data analysis specialists of the applicant's company and by the expert community (the medical community, the industrial community, etc.).

The above-mentioned neural network comprises: a convolution component configured to transform the original X-ray image into a map of variables which encodes information about objects in the original X-ray image and transmit the map of variables to the Region Proposal Network (RPN) component; the RPN component configured to calculate predictions of relevant objects on the map of variables and transmit the map of variables and the calculated predictions to a classification component; the classification component configured to calculate a probability that the object found in the X-ray image is a background object, and transmit classified data to a regression component and an aggregation component; the regression component configured to determine the exact location of the found object in the image and transmit the processed data to the aggregation component; and the aggregation component configured to aggregate information of the data obtained from the classification component and the regression component. The aggregation yields summary data of a type of the found object.

The data post-processing unit 106 is configured to post-process and transmit the data to the data storage unit 107, to the display unit 108 for the data to be outputted to at least one user, and to the data interpretation unit 109. Said post-processing comprises calculating the result of matching the physical parameters of the found and processed object in accordance with the selected optimal weights. The process of selecting the optimal weights is specified in paragraph 20 of the present application. This unit draws the conclusion on the prediction of disease/pathology presence in percent, determines a disease class, type, benignity, and malignancy, and converts the same into a standardized form. When studying industrial facilities, it predicts risks and risk values when deviances from a norm are detected. Then, it transmits the relevant information to a result generation unit and an archive. In parallel, pathologies and diseases are automatically identified at a visual level, while obtaining their coordinates in the picture, and subsequently visually outputting the result and recording into the archive. The same happens when studying the industrial facilities.

The data storage unit 107 is configured to receive, transmit and store the X-ray images and their data. The unit 107 may be implemented as a permanent computer-readable medium comprising instructions which cause a processor to transmit, receive and store the above-mentioned data.

The display unit 108 is designed to decrypt the encrypted interpreted data and display the data processed by the above-mentioned data units to at least one user. One or more displays, such as CRT, LCD, plasma, touchscreen, projector, LED, OLED, etc., may be used as the unit 108.

The data interpretation unit 109 is configured to generate the interpreted data and encrypt the interpreted data. The interpreted data details the above-mentioned calculation of the results of the data post-processing unit. The interpretation involves determining the influence of the found objects of various classes on final-recommendation generation and highlighting the image regions which have most influenced a model prediction consisting in that this is an object of a certain class, with subsequent outputting the interpreted data by the display unit to at least one user. The interpretation means an alternative data interpretation with a significant proportion of visual and textual data and the use of complex ensemble architectures based on machine learning, for example, SHAP values, Lime. The data interpretation unit 109 is required to make the claimed system applicable for tasks with a high error cost, for example, oncology diagnostics. In general, the data interpretation unit 109 describes why a certain prediction of disease/pathology presence in percent has been derived, and why a particular disease class, type, benignity and malignancy has been determined. When interpreting the data, the unit 109 determines which characteristics of the X-ray image have influenced the determination of the physical data of the object under study, their classification, etc. The interpretation may be performed by analyzing logistic regression coefficients and calculating a gradient with respect to the input data of the X-ray image. The analysis of the logistic regression coefficients is a global interpretation, while the calculation of the gradient with respect to the input data of the X-ray image provides the location of objects and is a local interpretation. Additionally, it should be noted that the above-mentioned units (all or some) may also be implemented on the remote server 111.

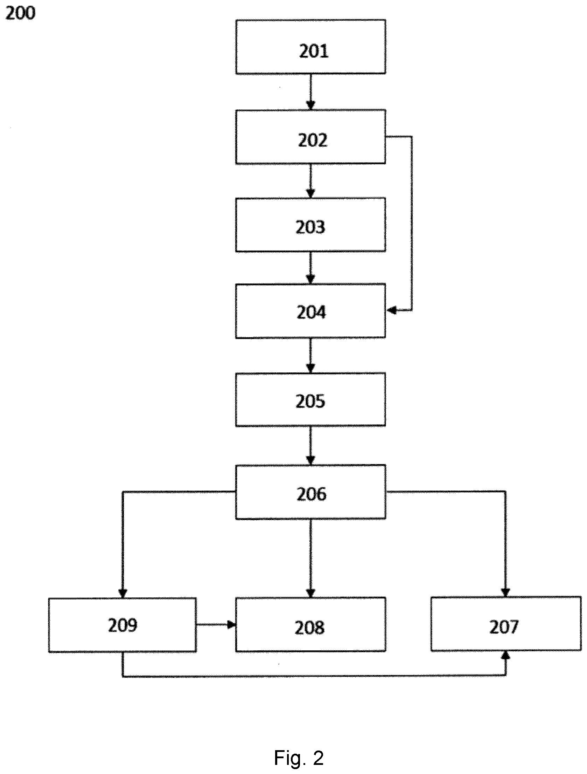

FIG. 2 shows a block diagram of steps for processing X-ray images.

At a step 201, the X-ray image files are downloaded into the system 100. The data may be downloaded both from external (relative to the system 100) sources and by means of devices capable of generating X-ray images. The process then proceeds to a step 202.

At the step 202, the X-ray images are pre-processed. If the processed data do not need to be transmitted to the remote server 111, then the process goes to a step 204; otherwise - to a step 203. The transmission of the processed data to the remote server at the step 202 or the absence of the need for this transmission is determined automatically by the administrator of the claimed system.

At the step 203, the processed X-ray images are compressed or unzipped, transmitted, or received between the remote server 111 and the compressing or unzipping unit 103.

At the step 204, at least one similar object is found (by the unit 104) in at least two pre-processed images and processed. After it is found, the process proceeds to a step 205.

At the step 205, the data is processed by the trainable neural network. At this step, the physical parameters of the found and processed objects are compared. As a result of this processing, the object is subjected to classification (e.g., mass lesion, benign tumor, malignant tumor, etc.). The process then goes to a step 206.

At the step 206, the post-processing of the data occurs (via the unit 106). As a result of this post-processing, the prediction of disease/pathology presence in percent is outputted, and a disease class, type, benignity, and malignancy are determined. The data are converted into a standardized form. In case of studying industrial facilities, the prediction of risks and their values is performed when deviations from a norm are detected. After the step 206, the process proceeds simultaneously to steps 207, 208, and 209.

At the step 207, the post-processed data and the data interpreted in accordance with the step 209 are stored. This storing may be performed both in the memory of the server 111 and in the memory of the user computing device, or in both segments.

At the step 208, the processed (in accordance with the steps 201, 202, 203, 204, 205, 206) data and/or the interpreted data (in accordance with the step 209) are displayed to at least one user.

At the step 209, the data interpretation occurs, according to which the calculation of the results of the data post-processing unit 106 is detailed by analyzing the logistic regression coefficients, calculating the gradient with respect to the input data and outputting the interpreted data via the display unit to the at least one user. After the interpretation procedure, the process simultaneously goes to the steps 207 and 208.

Although this invention has been shown and described with reference to certain embodiments thereof, those skilled in the art will appreciate that various changes and modifications may be made therein, without going beyond the actual scope of the invention.

## Claims

1. A system for processing an X-ray image and outputting a processing result to a user, comprising:
an X-ray image input unit configured to download X-ray image files containing metadata comprising information about an object or subject of an X-ray image and information about the X-ray image itself and transmit the downloaded files to an X-ray image pre-processing unit, as well as configured to encrypt the downloaded files if the files contain personal data of a person;
the X-ray image pre-processing unit configured to decrypt the encrypted downloaded files, process the X-ray image and transmit the pre-processed X-ray image to a compressing or unzipping unit, wherein said processing comprises: finding and capturing a relevant region of the X-ray image, removing noises from the captured relevant region of the X-ray image, the relevant region of the X-ray image being a region with the found object;
the compressing or unzipping unit configured to compress or unzip the pre-processed X-ray image for further transmission to an object finding unit;
the object finding unit for finding at least one similar object, configured to find the at least one similar object in at least two pre-processed images, process the at least one similar object and transmit the found and processed object to a data processing unit, said finding and processing comprise: finding the similar object in the images and superposing variable coordinates, distances and metrics on the images, the two pre-processed images being two pictures of different projections of a same human tissue or industrial facility under study;
the data processing unit configured to process the data by using a trainable neural network and transmit the processed data to a data post-processing unit, wherein said processing by using the neural network and its training comprise identifying patterns of the found and processed objects of a set of X-ray images and then identifying the region in the found and processed object based on the identified patterns;
the data post-processing unit configured to post-process and transmit the data to a data storage unit, a display unit and a data interpretation unit, wherein said post-processing comprises calculating a result of matching physical parameters of the identified region with the identified patterns in percentage terms and classifying the identified region;
the data interpretation unit configured to generate, encrypt and transmit interpreted data to the display unit, wherein the data interpretation unit is connected to a database of the data storage unit, the database comprising data of all classifications of the identified regions, wherein said interpreting comprises determining an influence of the found objects of various classes on final-recommendation generation and highlighting regions of the image which have most influenced a model prediction consisting in that this is an object of a certain class; and
the display unit configured to decrypt the encrypted interpreted data and output the decrypted interpreted data to at least one user.

2. The system of claim 1, wherein said finding and processing further comprise building a three-dimensional display of a shape of the found object, and said superposing the variable coordinates, distances and metrics on the images is performed in accordance with the three-dimensional display.

3. The system of claim 1, wherein the trainable neural network comprises:
a convolution component configured to transform the original X-ray image into a map of variables which encodes information about objects in the original X-ray image and transmit the map of variables to a region proposal network (RPN) component;
the RPN component configured to calculate predictions of relevant objects on the map of variables and transmit the map of variables and the calculated predictions to a classification component;
the classification component configured to calculate a probability that the object found in the X-ray image is a background object, and transmit classified data to a regression component and an aggregation component;
the regression component configured to determine an exact location of the found object in the image and transmit processed data to the aggregation component; and
the aggregation component configured to aggregate information of the data obtained from the classification component and the regression component, wherein the aggregation yields summary data of a type of the found object.

4. The system of claim 1, wherein the X-ray image has a format that may be at least one of: DICOM, JPEG, JPG, GIF, PNG.

5. The system of claim 1, wherein the X-ray image is an X-ray picture of human organs or tissues, or an X-ray picture of an industrial facility.

6. The system of claim 1, wherein the object has a physical parameter that is a kind of the object.
